# FASCICULE DE BREVET EUROPEEN

(11) **EP 1 412 049 B1**
(45) Date de publication et mention de la délivrance du brevet: **13.04.2005**
(21) Numéro de dépôt: 01972181.0
(22) Date de dépôt: 21.09.2001
(51) Int. Cl.: B01D 33/11, B01D 33/46, B01D 33/48, B01D 33/52, B01D 33/66, B01D 33/80

(54) **PROCEDE POUR ISOLER ET SECHER DES MICROPARTICULES (MICROSPHERES OU MICROCAPSULES) INITIALEMENT DISPERSEES OU SUSPENDUES EN PHASE LIQUIDE**
VERFAHREN ZUR ISOLIERUNG UND TROCKNUNG VON MIKROPARTIKELN ( MIKROSPHÄREN ODER MIKROKAPSELN ) AUS EINER FLÜSSIGEN PHASE
METHOD FOR ISOLATING AND DRYING MICROPARTICLES (MICROSPHERES OR MICROCAPSULES) INITIALLY DISPERSED OR SUSPENDED IN LIQUID PHASE

(30) Priorité: 21.09.2000 FR 0012027
(43) Date de publication de la demande: 28.04.2004
(73) Titulaire: LABORATOIRES DES PRODUITS ETHIQUES ETHYPHARM, 78550 Houdan (FR); MAINELAB, 49100 Angers (FR)
(72) Inventeur: RICHARD, Joel, F-49124 Saint Barthelemy d'Anjou (FR); ROMAIN, Patrice, F-76000 Rouen (FR)
(74) Mandataire: Texier, Christian
(86) Numéro de dépôt international: PCT/FR2001/002943
(87) Numéro de publication internationale: WO 2002/024305

(56) Documents cités:
- DE-A- 19 654 134
- DE-C- 505 887
- GB-A- 286 855
- US-A- 4 248 709

## Description

L'invention concerne la fabrication de microsphères et de microcapsules, et plus précisément la séparation de celles-ci à partir d'une phase liquide et leur séchage.

On sait que les microparticules, telles que les microsphères et les microcapsules à usage pharmaceutique, sont généralement produites au sein d'une phase liquide dont il s'agit ensuite de les extraire pour en faire des formes sèches solides administrables soit sous forme de poudres, soit sous forme de suspensions liquides reconstituées. Elles peuvent être préparées par les procédés connus de l'homme de l'art, telles que les méthodes d'émulsion-évaporation (ou extraction) de solvant ou les méthodes de conservation simple ou complexe. Afin de les séparer de la phase liquide, un procédé connu comprend l'étape de filtrer la préparation afin d'évacuer la plus grande partie de la phase liquide. Le filtrat qui en résulte forme un gâteau pâteux concentré de microparticules contenant un reste de phase liquide. On gratte et/ou racle alors ce gâteau pour le détacher du filtre et on place le gâteau dans une enceinte de lyophilisation. La lyophilisation permet d'extraire la phase liquide résiduelle pour l'obtention d'une masse de microsphères sèches, que l'on va ainsi pouvoir conserver de façon stable.

Toutefois, ce procédé présente de nombreux inconvénients au stade industriel. Tout d'abord, pour les formes pharmaceutiques de microparticules (microsphères ou microcapsules) destinées à être injectées (voie parentérale), le produit doit être stérile. Par conséquent, toutes les étapes de fabrication s'effectuent en salle à atmosphère contrôlée en classe 100 (stérile). Or, l'obtention de telles conditions dans des enceintes de travail de grand volume est très onéreuse. On s'efforce donc de confiner au maximum le produit pour chaque étape. Mais le transfert du produit de l'un à l'autre des appareils oblige à un passage en milieu ouvert. La stérilité est donc ou bien difficile ou bien coûteuse à assurer.

De plus, on constate que le procédé précité donne un rendement médiocre, car les différentes manipulations du produit conduisent à des pertes (sur le filtre, le lyophilisateur, dans le récipient de transfert,...).

Un but de l'invention est de faciliter la production de microparticules sèches stériles et d'en améliorer le rendement.

En vue de la réalisation de ce but, on prévoit selon l'invention un procédé pour séparer et sécher des microparticules initialement dispersées en phase liquide, comprenant les étapes consistant à :
- disposer une préparation comprenant des microparticules dispersées ou suspendues en phase liquide dans un récipient filtrant disposé à l'intérieur d'une enceinte ;
- filtrer une fraction de la phase liquide à travers le récipient dans l'enceinte ; et
- faire varier la température et la pression dans toute l'enceinte pour lyophiliser dans le récipient maintenu dans l'enceinte, la préparation filtrée.

Ainsi, l'exécution de la filtration et de la lyophilisation dans la même enceinte permet de réduire le volume à confiner. De plus, on supprime un transfert du produit entre ces deux étapes, qui était un facteur important de rupture de stérilité et de baisse de rendement. Il est donc plus facile et moins coûteux d'obtenir un produit sec et stérile.

Avantageusement, on met ou on maintient le récipient en mouvement durant une partie ou l'ensemble de l'étape de filtration.

Avantageusement, on met le récipient en mouvement durant une partie ou l'ensemble de l'étape de lyophilisation.

Ainsi, ce mouvement entraîne que durant ces étapes, le gâteau se répartit davantage et de façon plus homogène sur la surface interne du récipient. La couche de gâteau qui se constitue est plus fine et plus régulière, ce qui facilite l'extraction de la phase liquide. De plus, la filtration et/ou la lyophilisation sont plus rapides à effectuer.

Avantageusement, le mouvement comprend un mouvement de rotation.

La rotation accroît particulièrement ces avantages.

Avantageusement, la rotation s'effectue autour d'un axe de symétrie du récipient.

Avantageusement, l'axe de symétrie est un axe de symétrie de révolution du récipient.

Avantageusement, la rotation s'effectue autour d'un axe non vertical.

On obtient ainsi une répartition particulièrement régulière du gâteau sur la paroi du récipient, améliorant encore les avantages précités.

Avantageusement, l'axe de rotation fait avec l'horizontale un angle inférieur à 25°.

Avantageusement, le récipient comprend un tronçon profilé.

Avantageusement, le récipient comprend un tronçon cylindrique.

Avantageusement, on met la préparation en surpression durant l'étape de filtration.

Avantageusement, après l'étape de lyophilisation, on met l'intérieur de l'enceinte en surpression de gaz.

Grâce à cette surpression, une partie au moins du gâteau lyophilisé se sépare de la paroi, ce qui facilite sa récupération ultérieure.

Avantageusement, après l'étape de lyophilisation, on introduit des billes dans le récipient et on met le récipient en mouvement.

Ces billes fractionnent le gâteau et facilitent son extraction ultérieure.

Avantageusement, après l'étape de lyophilisation, on racle l'intérieur du récipient.

Avantageusement, après l'étape de lyophilisation, on extrait les microparticules hors du récipient par gravité.

On prévoit également selon l'invention un dispositif pour isoler des microparticules initialement dispersées ou suspendues en phase liquide, comportant un récipient de filtration, une enceinte et des moyens pour modifier la température et la pression dans toute l'enceinte, dans lequel le récipient s'étend dans l'enceinte.

Le dispositif selon l'invention pourra en outre présenter au moins l'une des caractéristiques suivantes :
- le récipient de filtration est monté mobile dans l'enceinte ;
- le récipient de filtration est monté mobile à rotation ;
- le récipient de filtration est monté mobile à rotation autour d'un axe de symétrie du récipient ;
- l'axe est un axe de symétrie de révolution du récipient ;
- le dispositif est agencé pour que l'axe de rotation soit incliné par rapport à la verticale ;
- le dispositif comprend des moyens pour modifier l'inclinaison de l'axe de rotation ;
- le récipient comprend une paroi de filtration cylindrique ;
- le dispositif comprend des moyens d'échange thermique de même forme qu'une partie du récipient et disposés coaxialement en regard de celle-ci ;
- le récipient présente une ouverture d'introduction et/ou d'évacuation située à une extrémité axiale du récipient ; et
- le dispositif comporte des moyens de raclage d'une face interne du récipient.

D'autres caractéristiques et avantages de l'invention apparaîtront encore dans la description suivante d'un mode préféré de réalisation donné à titre d'exemple non limitatif.

Aux dessins annexés :
- la figure 1 est une vue en coupe illustrant un exemple des principaux éléments du dispositif selon l'invention ;

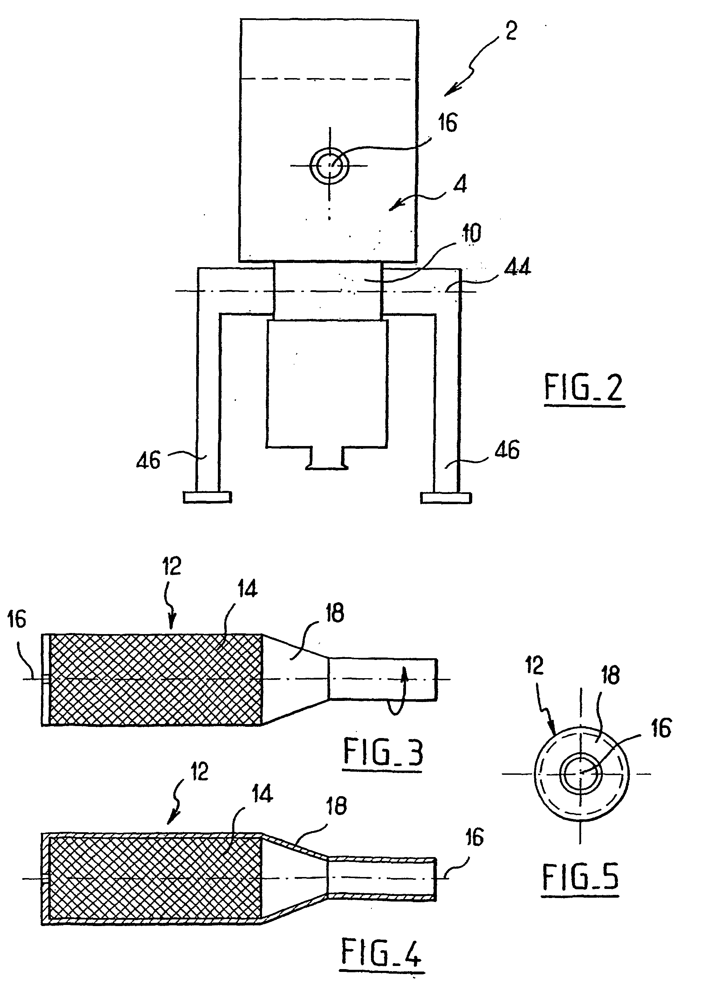

- la figure 2 est une vue de gauche du dispositif de la figure 1 ;
- la figure 3 est une vue en élévation du récipient filtrant du dispositif de la figure 1 ;
- la figure 4 est une vue en coupe axiale du récipient filtrant de la figure 3 ;
- la figure 5 est une vue de droite du récipient de la figure 3 ; et
- les figures 6 à 9 sont des vues analogues à la figure 1 illustrant différentes étapes successives de la mise en oeuvre du procédé selon l'invention.

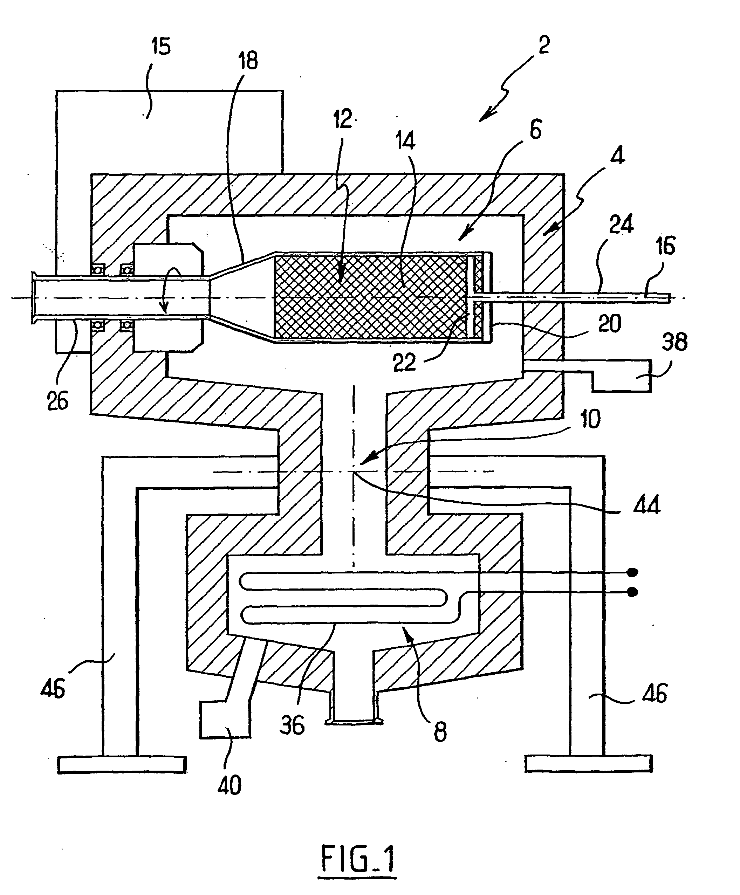

En référence à la figure 1, le dispositif 2 selon l'invention destiné à séparer et sécher des microparticules initialement en phase liquide, comporte une enceinte 4 présentant une chambre supérieure 6 et une chambre inférieure 8 dite « piège » ou condenseur. Les deux chambres s'étendent l'une au-dessus de l'autre et sont séparées l'une de l'autre par un étranglement 10 à travers lequel elles communiquent. Le dispositif comporte un récipient filtrant 12 s'étendant dans la chambre supérieure 6. Le récipient filtrant a un axe 16. Il est profilé suivant cet axe et présente une section transversale circulaire. Le récipient filtrant comporte ici une paroi filtrante cylindrique 14. Cette paroi présente des micro-orifices aptes à laisser passer la phase liquide, ici aqueuse, de la préparation contenant les microsphères, sans laisser échapper les microsphères. Cette paroi, connue en soi, peut être réalisée en différents matériaux :
- céramique creuse ;
- inox fritté ; ou
- matière synthétique frittée (par exemple polyéthylène).

Alternativement, la paroi 14 peut comprendre une couche rigide perforée avec des orifices de grand diamètre et intérieurement une membrane fine supportée par celle-ci. La membrane peut être une toile d'inox, d'acétate de cellulose, de nylon, etc.

Le choix du matériau de la paroi 14 se fera en fonction des caractéristiques des microsphères à récupérer (coût, dimensions, compatibilité avec le matériau).

Le récipient comporte à une extrémité axiale de la paroi, à gauche sur la figure 1, un tronçon tronconique 18, par exemple en acier inoxydable. La section la plus large du tronçon 18 est raccordée à la paroi cylindrique 14. La paroi cylindrique 14 a ici une longueur de 25 cm environ. La liaison entre la paroi 14 et le tronçon 18 peut être assurée par vissage, bridage ou soudage. L'autre extrémité axiale du récipient est obturée par une culasse 20 en forme de disque.

Le récipient 12 comprend un piston racleur 22 en forme de disque d'axe 16 ayant un plus grand diamètre voisin du diamètre de la face interne de la paroi cylindrique 14. Ce racleur est relié à une tige d'actionnement rectiligne 24, confondue avec l'axe 16 et traversant le centre de la culasse 20. Le racleur est mobile à coulissement sur toute la longueur de la paroi 14.

Le récipient filtrant 12 est monté mobile à rotation dans la chambre supérieure 6 par rapport à l'enceinte 4, autour de son axe 16. La tige 24 traverse la paroi de l'enceinte 4 de façon étanche pour pouvoir être manoeuvrée depuis l'extérieur de l'enceinte.

Le guidage en rotation et le support du récipient 12 sont assurés par un arbre creux 26 de forme cylindrique, d'axe 16, traversant la paroi de l'enceinte pour se raccorder à l'extrémité étroite du tronçon 18. L'intérieur de l'arbre 26 débouche ainsi dans le récipient 12. Des roulements à billes font office de palier entre la paroi de l'embase et l'arbre. Des moyens d'étanchéité sont prévus à cet endroit pour isoler l'extérieur et l'intérieur de l'enceinte.

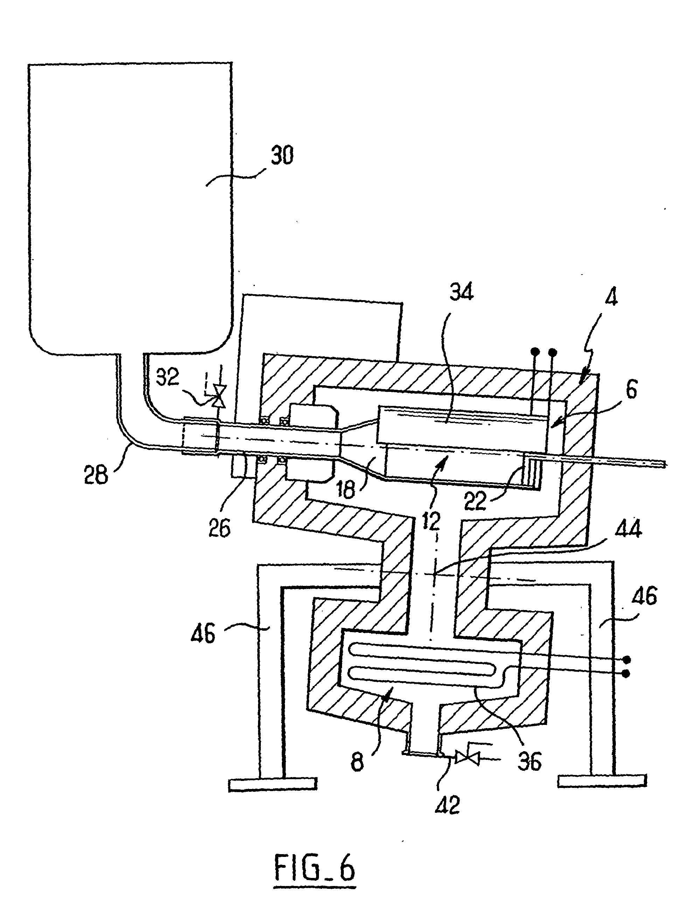

En référence à la figure 6, l'extrémité de l'arbre 26 la plus éloignée du récipient 12 peut être connectée à une conduite coudée 28 de même diamètre, elle-même en communication avec un réacteur 30 pour la préparation de microsphères. De la sorte, l'intérieur de l'enceinte ne communique avec le réacteur que via le récipient, précisément via les orifices de sa paroi 14. Le dispositif comporte une vanne 32 permettant de couper la communication entre l'arbre 26 et la conduite 28 afin d'isoler l'intérieur de l'enceinte à l'égard du réacteur 30 de façon étanche.

En référence à la figure 1, le dispositif comporte un moteur 15 apte à entraîner en rotation autour de l'axe 16 l'arbre 26 avec le récipient 14.

En référence à la figure 6, le dispositif comporte un serpentin d'échange thermique 34 formé par une canalisation en communication de fluide à l'extérieur de l'enceinte avec un groupe de production de chaud ou de froid (de - 60°C à + 40°C). Le serpentin 34 est conformé en demi-cylindre pour former une demi-coque. Son diamètre interne est un peu supérieur au diamètre externe de la paroi 12. Le serpentin 34 s'étend dans la chambre supérieure 6 en regard du demi-cylindre supérieur de la paroi 14, qu'il cache ainsi. Dans le piège 8, le dispositif comporte un serpentin de condenseur 36 en communication de fluide à l'extérieur de l'enceinte avec un groupe de production de froid pour maintenir le piège par exemple à - 60°C.

Les deux serpentins sont parcourus par des fluides caloporteurs adaptés.

Le dispositif comporte en outre des moyens 38 pour introduire de l'azote liquide dans la chambre supérieure 6 en vue de la congélation rapide avant la lyophilisation. Il comporte également une pompe 40, par exemple à palette, pour réaliser un vide poussé dans toute l'enceinte 4 en vue de la lyophilisation.

En référence à la figure 6, l'extrémité inférieure du piège 8 est obturable à volonté par une vanne d'étanchéité 42.

L'ensemble formé par l'enceinte et les éléments qu'elle contient est monté mobile en rotation autour d'un axe horizontal 44 perpendiculaire au plan de la figure 1 et passant géométriquement par l'étranglement 10. Le dispositif comporte à cette fin des moyens de support 46 soutenant l'enceinte sur le soubassement en permettant cette rotation. Cette rotation permet de modifier l'inclinaison de l'axe 16 par rapport à l'horizontale.

L'enceinte est mécano-soudée, en acier inoxydable et doublée d'une enveloppe calorifugée. L'intérieur est lisse avec des angles arrondis et sans zone de rétention pour un nettoyage facile.

A l'aide de ce dispositif, le procédé selon l'invention est en l'espèce mis en oeuvre de la façon suivante pour la fabrication de microsphères à usage pharmaceutique.

En référence à la figure 6, la conduite 26 est reliée au réacteur 30 par la bride étanche et rotative prévue à cet effet. Le piston racleur 22 est en position reculée.

On injecte à partir du réacteur 30 de la vapeur sous pression à 120° C pendant 20 mn pour stériliser l'ensemble des éléments (récipient, enceinte,...). Les condensats éventuels sont récupérés au bas du piège 8.

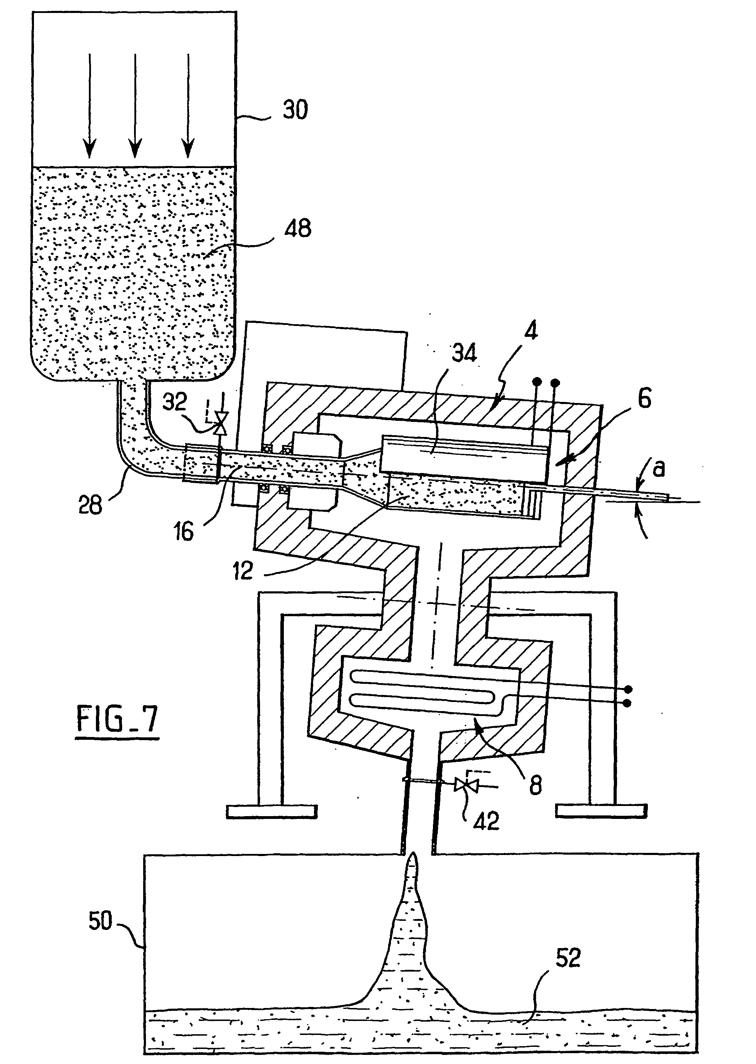

En référence à la figure 7, le récipient 14 revenu à température ambiante est alors mis en rotation. L'inclinaison de l'enceinte est choisie pour que l'angle a entre l'axe 16 et l'horizontale soit de 5°, le tronçon 18 formant l'amont du récipient.

Le réacteur 30 contient une dispersion 48 de microsphères en suspension dans une phase aqueuse. Il pourra s'agir par exemple d'une dispersion aqueuse 48 de microparticules de copolymère d'acide lactique et d'acide glycolique (PLGA) de diamètre moyen 50 µm, préparé par émulsion / extraction de solvant. Cette dispersion est mise sous pression puis envoyée dans le récipient 12. Sous l'effet de la pression et de la gravité, le liquide parcourt la conduite 28, l'arbre 26, le récipient filtrant 12 (dans lequel la légère pente supprime les zones de rétention) puis traverse la paroi 14 et s'écoule jusqu'au bas du piège 8. La vanne 42 étant ouverte et connectée à un réservoir 50, on récupère le liquide 52 dans ce dernier. Il demeure dans le récipient 14 un gâteau pâteux de microsphères réparti sur toute la face cylindrique interne de la paroi 12 en une couche fine sous l'effet de la rotation.

Ensuite, l'intérieur de l'enceinte est isolé de façon étanche de l'extérieur par la fermeture des deux vannes. Puis la machine est déconnectée du réacteur 30 et du réservoir 50.

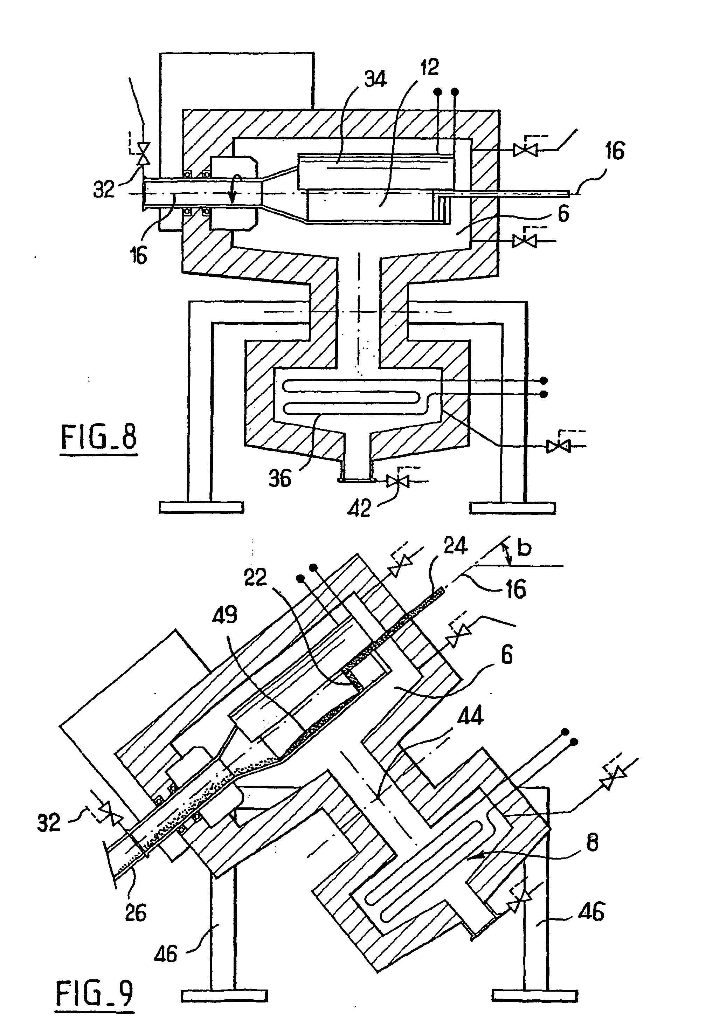

En référence à la figure 8, on modifie l'inclinaison de l'axe 16 pour qu'il soit horizontal. On envoie dans la chambre supérieure 6 de l'azote liquide pour porter tout l'intérieur de l'enceinte à - 60°C et opérer une congélation rapide du gâteau de microsphères. Le récipient 12 est toujours en rotation. Les deux chambres 6, 8 (c'est-à-dire tout l'intérieur de l'enceinte) sont ensuite mises sous vide poussé au moyen de la pompe 40.

On débute alors la phase de lyophilisation. Pour cela, on maintient le piège 8 à - 60°C au moyen du serpentin 36, pendant qu'au moyen du serpentin 34 en demi-coque, on ramène progressivement la température de la chambre supérieure 6 de - 60°C à + 40°C durant une durée adaptée. Le récipient demeure en rotation.

A l'issue de l'étape de lyophilisation, le liquide a été extrait du gâteau et s'est cristallisé dans le piège 8. Le gâteau de microsphères dans le récipient filtrant 12 est maintenant sec.

En référence à la figure 9, on incline l'enceinte (dans le sens antihoraire sur la figure 7) de sorte que l'axe 16 fait maintenant un angle de 40° avec l'horizontale, le tronçon 18 formant cette fois la partie aval du récipient. On ramène l'intérieur de l'enceinte à température ambiante en ouvrant la vanne 32 et en la raccordant à un tamis. Pour extraire le gâteau 49 du récipient, on injecte par impulsions dans la chambre supérieure 6 de l'air comprimé pour décoller le gâteau de la paroi 14, ce qui entraîne l'écoulement d'au moins une partie du gâteau par gravité jusqu'au tamis. Puis on racle l'intérieur du récipient d'amont en aval avec le piston 22 pour achever de décoller et/ou d'évacuer le gâteau résiduel.

On pourra en outre ou alternativement prévoir d'introduire des billes dans le récipient en rotation afin de fractionner le gâteau et le décoller de la paroi sous l'effet du mouvement des billes retenues dans le récipient.

On voit donc que le procédé selon l'invention regroupe la filtration et le séchage dans le même récipient et la même enceinte, sans intervention humaine sur le produit, en particulier sans transfert de récipient.

Comme on l'a vu, l'installation peut être préalablement connectée au réacteur pour une stérilisation initiale (procédé sous pression de vapeur). Dès lors la conduite des opérations de séparation - lyophilisation - récupération, pourra se faire dans des conditions strictement confinées apportant toute l'assurance de qualité de stérilité du produit.

La nature cylindrique du système de filtration, mis en rotation, permet une répartition uniforme du gâteau de microsphères, élément favorable pour l'obtention d'une lyophilisation optimale.

De plus, la surface développée de la forme cylindrique permet d'obtenir des épaisseurs de gâteau réduites par rapport à des filtres à surface plane. De plus, l'installation est plus compacte par rapport à un lyophilisateur à étagère.

Le gâteau de produit lyophilisé présentant fréquemment une certaine cohésion en fin de lyophilisation, le système de raclage permet d'évacuer le lyophilisat sans intervention manuelle, manipulation pourtant obligatoire pour la récupération de produit séché en plateaux (au moyen d'un lyophilisateur à étagère ou d'une armoire de séchage).

Le procédé selon l'invention évite également de rincer le gâteau avec une grande quantité d'eau, comme cela est parfois le cas dans des procédés connus, ce qui présente l'inconvénient d'amorcer la libération du produit encapsulé dans les microsphères.

Bien entendu, on pourra apporter à l'invention de nombreuses modifications sans sortir du cadre de celle-ci. L'invention pourra être appliquée à la production de microsphères ou de microcapsules dans les domaines pharmaceutiques humain ou vétérinaire, la cosmétique ainsi que d'autres domaines industriels tels que le textile, la chimie fine, l'imprimerie, etc.

Le récipient filtrant pourra avoir une forme de révolution autre que celle d'un cylindre. Il pourrait par exemple s'agir d'une sphère, qui cependant ne permet pas le raclage.

Le récipient filtrant pourra avoir une forme autre qu'une forme de révolution autour d'un axe, par exemple une forme profilée à section polygonale.

Par ailleurs, on pourra modifier les paramètres opératoires (inclinaison, température, durées, etc).

A titre de second exemple, la même procédure d'isolation et de séchage des microparticules peut-être appliquée à une dispersion aqueuse de microcapsules gélatine - alginate réticulées contenant une matière active dispersée dans une phase huileuse, initialement préparées par coacervation complexe dans le réacteur 30 et présentant une taille moyenne de 100 µm.

## Revendications

1. Procédé pour séparer et sécher des microparticules initialement dispersées en phase liquide, **caractérisé en ce qu'**il comprend les étapes consistant à :
- disposer une préparation (48) comprenant des microparticules dispersées ou suspendues en phase liquide dans un récipient filtrant (12) disposé à l'intérieur d'une enceinte (6) ;
- filtrer une fraction de la phase liquide (52) à travers le récipient dans l'enceinte ; et
- faire varier la température et la pression dans toute l'enceinte pour lyophiliser dans le récipient maintenu dans l'enceinte, la préparation filtrée.

2. Procédé selon la revendication 1, **caractérisé en ce que** le récipient filtrant (12) est en mouvement durant une partie ou l'ensemble de l'étape de filtration.

3. Procédé selon la revendication 1 ou 2, **caractérisé en ce que** le récipient filtrant (12) est en mouvement durant une partie ou l'ensemble de l'étape de lyophilisation.

4. Procédé selon la revendication 2 ou 3, **caractérisé en ce que** le mouvement comprend un mouvement de rotation, en particulier autour d'un axe de symétrie (16) du récipient (12), de préférence un axe de symétrie de révolution du récipient (12).

5. Procédé selon l'une quelconque des revendications 1 à 4, **caractérisé en ce que** le récipient comprend un tronçon cylindrique (14).

6. Procédé selon l'une quelconque des revendications 1 à 5, **caractérisé en ce qu'**on met la préparation (48) en surpression durant l'étape de filtration.

7. Procédé selon l'une quelconque des revendications 1 à 6, **caractérisé en ce qu'**après l'étape de lyophilisation, on met l'intérieur de l'enceinte (6) en surpression de gaz.

8. Procédé selon l'une quelconque des revendications 1 à 7, **caractérisé en ce qu'**après l'étape de lyophilisation, on introduit des billes dans le récipient (12) et on met le récipient en mouvement.

9. Procédé selon l'une quelconque des revendications 1 à 8, **caractérisé en ce qu'**après l'étape de lyophilisation, on racle l'intérieur du récipient (12).

10. Dispositif (2) pour séparer et sécher des microparticules initialement dispersées ou suspendues en phase liquide, comportant un récipient de filtration (12), une enceinte (6) et des moyens (34, 36, 38, 40) pour modifier la température et la pression dans toute l'enceinte à des fins de lyophilisation dans le dispositif, **caractérisé en ce que** le récipient de filtration (12) s'étend dans l'enceinte (6).

11. Dispositif selon la revendication 10, **caractérisé en ce que** le récipient (12) est monté mobile dans l'enceinte (6), en particulier mobile à rotation autour d'un axe de symétrie (16) du récipient.

12. Dispositif selon la revendication 11, **caractérisé en ce qu'**il comprend des moyens (44, 46) pour modifier l'inclinaison de l'axe de rotation (16).

13. Dispositif selon l'une quelconque des revendications 10 à 12, **caractérisé en ce que** le récipient de filtration (12) comprend une paroi de filtration cylindrique (14).

14. Dispositif selon l'une quelconque des revendications 10 à 13, **caractérisé en ce qu'**il comprend des moyens d'échange thermique (34) de même forme qu'une partie (14) du récipient (12) et disposés coaxialement en regard de celle-ci.

15. Dispositif selon l'une quelconque des revendications 10 à 14, **caractérisé en ce que** le récipient (12) présente une ouverture d'introduction et/ou d'évacuation située à une extrémité axiale du récipient.

16. Dispositif selon l'une quelconque des revendications 10 à 15, **caractérisé en ce qu'**il comporte des moyens (22) de raclage d'une face interne du récipient (12).

## Patentansprüche

1. Verfahren zum Abtrennen und Trocknen von Mikropartikeln, die anfänglich in einer flüssigen Phase dispergiert vorliegen, **dadurch gekennzeichnet, dass** es die Schritte umfasst, bestehend aus:
- Vorlegen einer Präparation (48), welche in einer flüssigen Phase dispergierte oder suspendierte Mikropartikel umfasst, in einem Behälter mit Filtrationsfunktion (12), welcher im Inneren einer Kammer (6) angeordnet ist;
- Filtrieren eines Teils der flüssigen Phase (52) durch den Behälter in der Kammer hindurch; und
- Variierenlassen der Temperatur und des Drucks in der gesamten Kammer, um in dem in der Kammer befindlichen Behälter die filtrierte Präparation zu lyophilisieren.

2. Verfahren nach Anspruch 1, **dadurch gekennzeichnet, dass** der Behälter mit Filtrationsfunktion (12) sich während eines Teils oder der Gesamtheit des Filtrationsschritts in Bewegung befindet.

3. Verfahren nach Anspruch 1 oder 2, **dadurch gekennzeichnet, dass** der Behälter mit Filtrationsfunktion (12) sich während eines Teils oder der Gesamtheit des Lyophilisationsschritts in Bewegung befindet.

4. Verfahren nach Anspruch 2 oder 3, **dadurch gekennzeichnet, dass** die Bewegung eine Rotationsbewegung, insbesondere um eine Symmetrieachse (16) des Behälters (12), vorzugsweise eine Symmetrierotationsachse des Behälters (12), herum umfasst.

5. Verfahren nach einem der Ansprüche 1 bis 4, **dadurch gekennzeichnet, dass** der Behälter einen zylindrischen Abschnitt (14) umfasst.

6. Verfahren nach einem der Ansprüche 1 bis 5, **dadurch gekennzeichnet, dass** man die Präparation (48) während des Filtrationsschritts unter Überdruck setzt.

7. Verfahren nach einem der Ansprüche 1 bis 6, **dadurch gekennzeichnet, dass** man nach dem Lyophilisationsschritt das Innere der Kammer (6) unter Gas-Überdruck setzt.

8. Verfahren nach einem der Ansprüche 1 bis 7, **dadurch gekennzeichnet, dass** man nach dem Lyophilisationsschritt Kugeln in den Behälter (12) einfüllt und man den Behälter in Bewegung versetzt.

9. Verfahren nach einem der Ansprüche 1 bis 8, **dadurch gekennzeichnet, dass** man nach dem Lyophilisationsschritt das Innere des Behälters (12) abkratzt.

10. Vorrichtung (2) zum Abtrennen und Trocknen von Mikropartikeln, die anfänglich in einer flüssigen Phase dispergiert oder suspendiert sind, umfassend einen Filtrationsbehälter (12), eine Kammer (6) und Mittel (34, 36, 38, 40) zur Modifizierung der Temperatur und des Drucks in der gesamten Kammer zu Zwecken einer Lyophilisation in der Vorrichtung, **dadurch gekennzeichnet, dass** der Filtrationsbehälter (12) sich in der Kammer (6) erstreckt.

11. Vorrichtung nach Anspruch 10, **dadurch gekennzeichnet, dass** der Behälter (12) in der Kammer (6) beweglich, insbesondere beweglich bezüglich einer Rotation um eine Symmetrieachse (16) des Behälters montiert ist.

12. Vorrichtung nach Anspruch 11, **dadurch gekennzeichnet, dass** sie Mittel (44, 46) umfasst, um die Neigung der Rotationsachse (16) zu modifizieren.

13. Vorrichtung nach einem der Ansprüche 10 bis 12, **dadurch gekennzeichnet, dass** der Filtrationsbehälter (12) eine zylindrische, der Filtration dienende Wand (14) umfasst.

14. Vorrichtung nach einem der Ansprüche 10 bis 13, **dadurch gekennzeichnet, dass** sie Wärmeaustauschmittel (34) von der gleichen Form wie ein Abschnitt (14) des Behälters (12), die coaxial in Bezug auf jenen angeordnet sind, umfasst.

15. Vorrichtung nach einem der Ansprüche 10 bis 14, **dadurch gekennzeichnet, dass** der Behälter (12) eine Einführ- und/oder Evakuierungsöffnung, welche sich an einem axialen Ende des Behälters befindet, aufweist.

16. Vorrichtung nach einem der Ansprüche 10 bis 15, **dadurch gekennzeichnet, dass** sie Mittel (22) zum Abkratzen einer inneren Fläche des Behälters (12) umfasst.

## Claims

1. Process for separating and drying microparticles initially dispersed in a liquid phase, **characterized in that** it comprises the steps consisting in:
- placing a preparation (48) comprising microparticles dispersed or suspended in a liquid phase in a filtration container (12) placed inside a chamber (6);
- filtering a fraction of the liquid phase (52) through the container in the chamber; and
- varying the temperature and the pressure throughout the chamber in order to freeze dry the filtered preparation in the container maintained in the chamber.

2. Process according to Claim 1, **characterized in that** the filtration container (12) is in motion during part or all of the filtration step.

3. Process according to Claim 1 or 2, **characterized in that** the filtration container (12) is in motion during part or all of the freeze-drying step.

4. Process according to Claim 2 or 3, **characterized in that** the motion comprises a rotational motion, in particular about an axis (16) of symmetry of the container (12), preferably an axis of symmetry of revolution of the container (12).

5. Process according to any one of Claims 1 to 4, **characterized in that** the container has a cylindrical portion (18).

6. Process according to any one of Claims 1 to 5, **characterized in that** the preparation (48) is put under an overpressure during the filtration step.

7. Process according to any one of Claims 1 to 6, **characterized in that**, after the freeze-drying step, the inside of the chamber (6) is put under a gas overpressure.

8. Process according to any one of Claims 1 to 7, **characterized in that**, after the freeze-drying step, balls are introduced into the container (12) and the container is set in motion.

9. Process according to any one of Claims 1 to 8, **characterized in that**, after the freeze-drying step, the inside of the container (12) is scraped.

10. Apparatus (2) for separating and drying microparticles initially dispersed or suspended in a liquid phase, comprising a filtration container (12), a chamber (6) and means (34, 36, 38, 40) for modifying the temperature and the pressure throughout the chamber for the purposes of freeze-drying in the apparatus, **characterized in that** the filtration container (12) lies in the chamber (6).

11. Apparatus according to Claim 10, **characterized in that** the container (12) is mounted so as to move in the chamber (6), in particular it is mounted so as to move in rotation about an axis (16) of symmetry of the container.

12. Apparatus according to Claim 11, **characterized in that** it includes means. (44, 46) for modifying the angle of inclination of the rotation axis (16).

13. Apparatus according to any one of Claims 10 to 12, **characterized in that** the filtration container (12) has a cylindrical filtration wall (14).

14. Apparatus according to any one of Claims 10 to 13, **characterized in that** it includes heat exchange means (34) that have the same shape as a part (14) of the container (12) and are placed coaxially with respect to said part.

15. Apparatus according to any one of Claims 10 to 14, **characterized in that** the container (12) has, at an axial end thereof, an opening for introduction and/or removal.

16. Apparatus according to any one of Claims 10 to 15, **characterized in that** it includes means (22) for scraping an internal face of the container (12).
